# EUROPEAN PATENT SPECIFICATION

(11) **EP 3 908 841 B1**
(45) Date of publication and mention of the grant of the patent: **08.03.2023**
(21) Application number: 20700163.7
(22) Date of filing: 10.01.2020
(51) Int. Cl.: G01N 33/68

(54) **IN VITRO METHOD FOR THE DIAGNOSIS OR PROGNOSIS OF NEURODEGENERATIVE DISORDERS**
IN-VITRO-VERFAHREN ZUR DIAGNOSE ODER PROGNOSE VON NEURODEGENERATIVEN ERKRANKUNGEN
PROCÉDÉ IN VITRO DE DIAGNOSTIC OU DE PRONOSTIC DE TROUBLES NEURODÉGÉNÉRATIFS

(30) Priority: 10.01.2019 EP 19382013
(43) Date of publication of application: 17.11.2021
(73) Proprietor: Fundación Biomédica Galicia Sur (FBGS), 36312 Vigo (ES); Servizo Galego de Saúde (SERGAS), 15703 Santiago de Compostela (ES); Universidad de Vigo, 36310 Vigo (ES)
(72) Inventor: SPUCH CALVAR, Carlos, 36312 Vigo (ES); RIVERA BALTANÁS, Tania, 36312 Vigo (ES); OLIVARES DÍAZ, Jose Manuel, 15703 Santiago de Compostela (ES); CORREA DUARTE, Miguel Ángel, 36310 Vigo (ES); PEREZ LORENZO, Moisés, 36310 Vigo (ES); SALGUEIRIÑO MACEIRA, Verónica, 36310 Vigo (ES); SOUSA CASTILLO, Ana, 36310 Vigo (ES); BLANCO FORMOSO, María, 36310 Vigo (ES)
(74) Representative: Hoffmann Eitle
(86) International application number: PCT/EP2020/050544
(87) International publication number: WO 2020/144327

(56) References cited:
- WO-A1-2014/144605
- SUZUKI K ET AL: "Reduced expression of apolipoprotein E receptor type 2 in peripheral blood lymphocytes from patients with major depressive disorder", PROGRESS IN NEURO-PSYCHOPHARMACOLOGY & BIOLOGICAL PSYCHIATRY, ELSEVIER, GB, vol. 34, no. 6, 16 August 2010 (2010-08-16), pages 1007-1010, XP027171272, ISSN: 0278-5846 [retrieved on 2010-05-20]
- HUI WANG ET AL: "Involvement of chronic unpredictable mild stress-induced hippocampal LRP1 up-regulation in microtubule instability and depressive-like behavior in a depressive-like adult male rat model", PHYSIOLOGY AND BEHAVIOR, vol. 215, 23 November 2019 (2019-11-23), page 112749, XP055666193, GB ISSN: 0031-9384, DOI: 10.1016/j.physbeh.2019.112749

## Description

### FIELD OF THE INVENTION

The present invention refers to the medical field. Particularly, the present invention is focused on an *in vitro* method for the diagnosis or prognosis of neurodegenerative disorders. The method comprises measuring the level of expression or the concentration level of at least a lipoprotein receptor-related protein (LRP) or a fragment thereof.

### STATE OF THE ART

Lipoprotein receptor-related proteins, low density lipoprotein receptor-related proteins (HGNC) or prolow-density lipoprotein receptor-related protein (UniProt), abbreviated LRP, are a group of proteins that include: LRP1, LRP1B, LRP2 (megalin), LRP3, LRP4, LRP5, LRP6, LRP8, LRP10, LRP11 and LRP12.

LRP1, also known as alpha-2-macroglobulin receptor (A2MR), apolipoprotein E receptor (APOER) or cluster of differentiation 91 (CD91), is a protein forming a receptor found in the plasma membrane of cells involved in receptor-mediated endocytosis. In humans, the LRP1 protein is encoded by the LRP1 gene. LRP1 is also a key signalling protein and, thus, involved in various biological processes, such as lipoprotein metabolism and cell motility, and diseases, such as neurodegenerative diseases, atherosclerosis, and cancer.

The LRP1 gene encodes a 600 kDa precursor protein that is processed by furin in the trans-Golgi complex, resulting in a 515 kDa alpha-chain and an 85 kDa beta-chain associated non-covalently. As a member of the LDLR family, LRP1 contains cysteine-rich complement-type repeats, EGF (gene) repeats, β-propeller domains, a transmembrane domain, and a cytoplasmic domain. The cytoplasmic domain of LRP1 is the alpha-chain, which comprises four ligand-binding domains (numbered I-IV) containing two, eight, ten, and eleven cysteine-rich complement-type repeats, respectively. These repeats bind extracellular matrix proteins, growth factors, proteases, protease inhibitor complexes, and other proteins involved in lipoprotein metabolism. Of the four domains, II and IV bind the majority of the protein's ligands. The EGF repeats and β-propeller domains serve to release ligands in low pH conditions, such as inside endosomes, with the β-propeller postulated to displace the ligand at the ligand binding repeats. The transmembrane domain is the β-chain, which contains a 100-residue cytoplasmic tail. This tail contains two NPxY motifs that are responsible for the protein's function in endocytosis and signal transduction.

LRP1 is a member of the LDLR family and ubiquitously expressed in multiple tissues, though it is most abundant in vascular smooth muscle cells (SMCs), hepatocytes, and neurons. LRP1 plays a key role in intracellular signalling and endocytosis, which thus implicate it in many cellular and biological processes, including lipid and lipoprotein metabolism, protease degradation, platelet derived growth factor receptor regulation, integrin maturation and recycling, regulation of vascular tone, regulation of blood brain barrier permeability, cell growth, cell migration, inflammation, and apoptosis, as well as diseases such as neurodegenerative diseases, atherosclerosis, and cancer. To elaborate, LRP1 mainly contributes to regulation of protein activity by binding target proteins as a co-receptor, in conjunction with integral membrane proteins or adaptor proteins like uPA, to the lysosome for degradation. In lipoprotein metabolism, the interaction between LRP1 and APOE stimulates a signalling pathway that leads to elevated intracellular cAMP levels, increased protein kinase A activity, inhibited SMC migration, and ultimately, protection against vascular disease. While membrane-bound LRP1 performs endocytic clearance of proteases and inhibitors, proteolytic cleavage of its ectodomain allows the free LRP1 to compete with the membrane-bound form and prevent their clearance. Several sheddases have been implicated in the proteolytic cleavage of LRP1 such as ADAM10, ADAM12, ADAM17 and MT1-MMP. LRP1 is also continuously endocytosed from the membrane and recycled back to the cell surface. Though the role of LRP1 in apoptosis is unclear, it is required for tPA to bind LRP1 in order to trigger the ERK1/2 signal cascade and promote cell survival.

Low density lipoprotein-related protein 2 also known as LRP2 or megalin is a protein which in humans is encoded by the LRP2 gene.

LRP2 was identified as the antigen of rat experimental membranous nephropathy (Heyman nephritis) and originally named gp330 and subsequently megalin and later LRP2. LRP2/megalin is a multiligand binding receptor found in the plasma membrane of many absorptive epithelial cells. LRP2/megalin is a member of a family of receptors with structural similarities to the low density lipoprotein receptor (LDLR). LRP2/megalin functions to mediate endocytosis of ligands leading to degradation in lysosomes or transcytosis. LRP2/megalin can also form complexes with cubilin: those complexes are able to reabsorb several molecules and can be inhibited by sodium maleate. LRP2 is expressed in epithelial cells of the thyroid (thyrocytes), where it can serve as a receptor for the protein thyroglobulin (Tg).

Having this prior art in mind, the present invention is focused on the provision of a panel of biomarkers precisely elected among the above cited LRP proteins or fragments thereof, aimed at diagnosing specific neurological and neurodegenerative diseases, for example: major depression, multiple sclerosis or Alzheimer disease.

### DESCRIPTION OF THE INVENTION

The present invention is directed to an *in vitro* method for the diagnosis or prognosis of neurodegenerative disorders. The method comprises measuring the level of expression or the concentration level of at least a LRP protein or a fragment thereof.

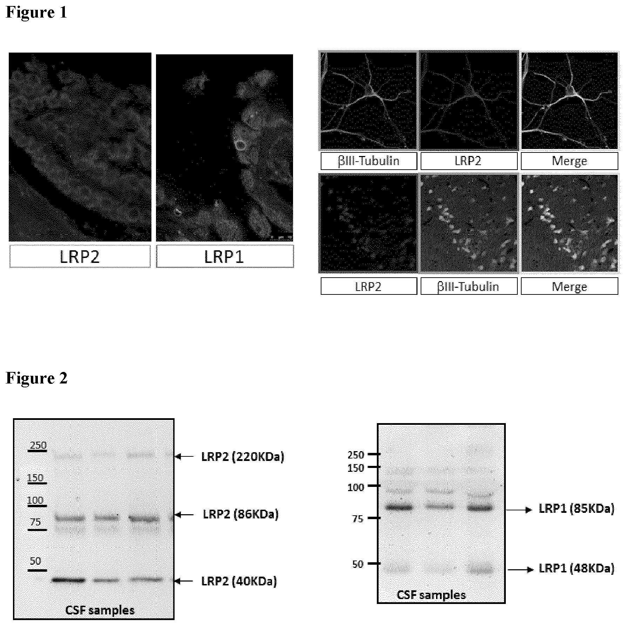

Among all the possible candidate biomarker, the inventors of the present invention focused their efforts on demonstrating the potential use of LRP proteins, or fragments thereof, in the diagnosis or prognosis of neurodegenerative disorders (**Figure 1**).

Particularly, the first embodiment of the present invention refers to an *in vitro* method for the diagnosis or prognosis of neurodegenerative disorders in a subject, selected from the list consisting of major depression, multiple sclerosis or Alzheimer disease, said method comprising: measuring the level of expression or the concentration level of at least a LRP protein or a fragment thereof, in a biological sample obtained from the subject, preferably serum, plasma, whole blood or cerebrospinal fluid (CSF), wherein if a deviation or variation of the level of expression or the concentration level of the LRP protein or of a fragment thereof is identified as compared with the level measured in healthy controls, this is indicative that the subject is suffering from a neurodegenerative disorder selected from the list consisting of: major depression, multiple sclerosis or Alzheimer disease, or has a bad prognosis.

The second embodiment of the present invention refers to the *in vitro* use of a LRP protein or a fragment thereof for the diagnosis or prognosis neurodegenerative disorders selected from the list consisting of: major depression, multiple sclerosis or Alzheimer disease.

The third embodiment of the present invention refers to the use of a kit comprising reagents for the identification of the level of expression or the concentration level of LRP proteins or fragments thereof, for the diagnosis or prognosis of neurodegenerative disorders selected from the list consisting of major depression, multiple sclerosis or Alzheimer disease.

In a preferred embodiment, the LRP fragment is selected from the list consisting of:
- LRP1 48 KDa of SEQ ID NO: 1,
- LRP1 441,179 KDa of SEQ ID NO: 2,
- LRP 1 498 KDa of SEQ ID NO: 3,
- LRP1 515 KDa of SEQ ID NO: 4,
- LRP1 497,696 KDa of SEQ ID NO: 5,
- LRP1 293,591 KDa of SEQ ID NO: 6,
- pro-LRP1 of 504,276 KDa of SEQ ID NO:7,
- LRP2 220 KDa of SEQ ID NO: 8 or
- LRP1 85 KDa of SEQ ID NO: 11.

In a preferred embodiment, the LRP protein is selected from the list consisting of:
- LRP1 of SEQ ID NO: 9 or
- LRP2BP of SEQ ID NO: 10.

In a preferred embodiment, the neurodegenerative disorder is major depression and the method comprises measuring the level of expression or the concentration level of at least a LRP1 fragment selected from the list consisting of: LRP1 48 KDa of SEQ ID NO: 1, LRP 1 441, 179 KDa of SEQ ID NO: 2, LRP 1 498 KDa of SEQ ID NO: 3, LRP 1 515 KDa of SEQ ID NO: 4, LRP1 497,696 KDa of SEQ ID NO: 5, LRP1 293,591 of SEQ ID NO: 6 or pro-LRP1 of 504,276 KDa of SEQ ID NO:7, wherein if the level of expression or the concentration level of the LRP1 fragment is significantly lower as compared with the level measured in healthy controls, this is indicative that the subject is suffering from major depression or has a bad prognosis.

In a preferred embodiment, the neurodegenerative disorder is major depression and the method comprises: measuring the ratio of the level of expression or the concentration level of LRP1 of SEQ ID NO: 9/proLRP1 of SEQ ID NO: 7 wherein if this ratio level is significantly higher as compared with the level measured in healthy controls, this is indicative that the subject is suffering from major depression or has a bad prognosis.

In a preferred embodiment, the neurodegenerative disorder is Alzheimer disease and the method comprises: measuring the level of expression or the concentration level of LRP2 220 KDa of SEQ ID NO: 8 wherein if this level is significantly lower as compared with the level measured in healthy controls, this is indicative that the subject is suffering from Alzheimer disease or has a bad prognosis.

In a preferred embodiment, the neurodegenerative disorder is multiple sclerosis and the method comprises: measuring the level of expression or the concentration level of LRP1 85 KDa of SEQ ID NO: 11 or LRP1 48 KDa of SEQ ID NO: 1 wherein if this level is significantly higher as compared with the level measured in healthy controls, this is indicative that the subject is suffering from multiple sclerosis or has a bad prognosis.

In a preferred embodiment, the neurodegenerative disorder is multiple sclerosis and the method comprises measuring the level of expression or the concentration level of pro-LRP1 of 504,276 KDa of SEQ ID NO: 7 or the level of expression or the concentration level of LRP1 of SEQ ID NO: 9 wherein if this level is significantly higher as compared with the level measured in healthy controls, this is indicative that the subject is suffering from multiple sclerosis or has a bad prognosis.

The last embodiment on the present invention refers to a method for the treatment of a neurodegenerative disorder selected from the list consisting of: major depression, multiple sclerosis or Alzheimer disease, which comprise previous step of diagnosing the disorder by means of the diagnostic methods described above.

For the purpose of the present invention the following terms are defined:
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of'. Thus, the phrase "consisting of" indicates that the listed elements are required or mandatory, and that no other elements may be present.
- The wording "reference level of expression or the concentration level measured in healthy control subjects", "reference", "control healthy subject" or "control level", when referring to the level of expression or concertation level of LRP proteins or fragments thereof, refers to the level observed in patients not suffering from the neurodegenerative disease. A patient is likely to suffer from a neurodegenerative disease, with a given sensitivity and specificity, if there is a deviation of the expression level or the concentration level of LRP proteins or fragments thereof in the patient as compared with the reference level measured in healthy control subjects.
- The wording "expression level or concentration statistically higher/lower than the reference control level" refers to a situation in which the level of expression or the concentration of LRP proteins or fragments thereof in the patient suffering from a neurodegenerative disease is at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 times higher/lower than the level of expression or concentration of the LRP proteins or fragments thereof in a control healthy subject.
- "Neurodegenerative disease" refers to those diseases caused by neurodegeneration (i.e. the progressive loss of structure or function of neurons, including death of neurons). Many neurodegenerative diseases occur as a result of neurodegenerative processes. Such diseases result in a progressive degeneration and/or death of neuron cells. As research progresses, many similarities appear that relate these diseases to one another on a sub-cellular level. Discovering these similarities offers hope for therapeutic advances that could ameliorate many diseases simultaneously. There are many parallels between different neurodegenerative disorders including atypical protein assemblies as well as induced cell death. Neurodegeneration can be found in many different levels of neuronal circuitry ranging from molecular to systemic.
- "Prognostic method" refers to methods used to help predict, at least in part, the course of a disease. For example, a screening procedure can be carried out on a subject who has not previously been diagnosed with a neurodegenerative disorder, or does not show substantial disease symptoms, when it is desired to obtain an indication of the future likelihood that the subject will be afflicted with the neurodegenerative disorder and/or the age at which the subject is likely to develop the neurodegenerative disorder. In addition, a prognostic method can be carried out on a subject previously diagnosed with the neurodegenerative disorder or believed or suspected to have the neurodegenerative disorder, when it is desired to gain greater insight into how the disease will progress for that particular subject (e.g., the likelihood that a particular subject will respond favourably to a particular drug or other treatment, and/or when it is desired to classify or separate the neurodegenerative disorder patients into distinct and different subpopulations for the purpose of administering a particular type of treatment and/or conducting a clinical trial thereon). A prognostic method can also be used to determine whether and/or how well a subject will respond to a particular drug and/or other treatment. Bad prognosis means that the subject will be afflicted with the neurodegenerative disorder and there is little chance for recovery.
- "Diagnostic method" as used herein refers to methods carried out on a subject to determine if the subject has the neurodegenerative disorder. Such a subject can be someone having no known risk factors, or someone who may be at risk or has previously been determined to be at risk for a particular neurodegenerative disorder due to the presentation of symptoms or the results of a screening test or other type of diagnostic test.

### BRIEF DESCRIPTION OF THE FIGURES

**Figure** 1. Expression of LRP2 and LRP1 in the human choroid plexus (left) and LRP2 in neurons (right). Immunofluorescence with confocal microscopy in human tissues choroid plexus, culture of neurons and cerebral cortex.
**Figure 2****.** Soluble fragments of LRP1 (right) and LRP2 (left) in CSF samples. Western blot in CSF samples directed against LRP1 and LRP2 in its extracellular portion. Left part of the figure shows three LRP2 fragments: 220KDa, 86KDa and 40KDa. Right part of the figure shows soluble fragments identified for LRP1: 85KDa fragment corresponds to the B chain of the LRP1, and the 48KDa fragment identified for the first time in the present invention by mass spectrometry LC-ESI-MS / MS.

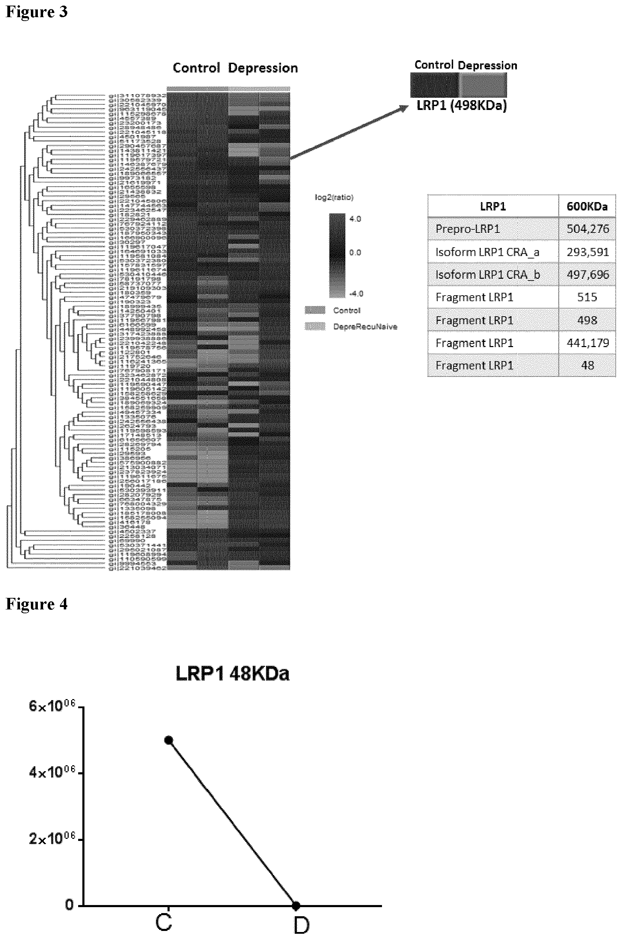

**Figure 3****.** Heat-map graph of the LC-MS / MS analysis in the Orbitrap ELITE (Thermo Scientific). 4 serum samples, two control serum samples and 2 samples from patients with Naïve Major Depressive Disorder (MDD) were analysed. 108 proteins that differentiate between control subjects and subjects with depression were identified. Among all these proteins, 7 fragments of LRP1 that change their expression or concentration between both groups were identified. The table on the right side shows the fragments identified showing a reduced level of expression or concentration in MDD.
**Figure 4****.** Quantification of the area under the curve (AUC) (Y axis) of the new 48KDa LRP1 fragment analysed in a patient with MDD identified by LC-MS / MS analysis. This fragment LRP1 48 KDa decreases its concentration in the serum of patients with MDD. C (Control). D (Depression).

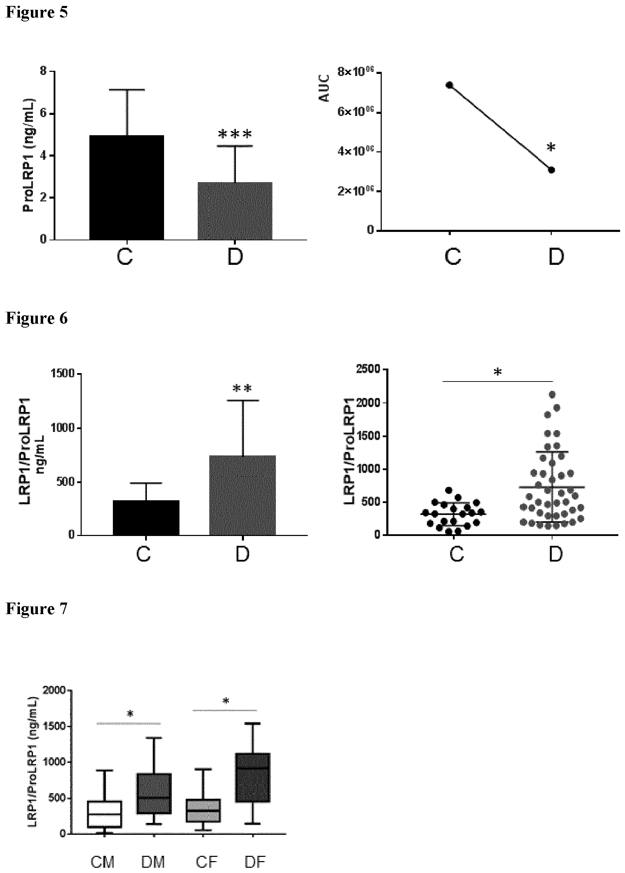

**Figure 5****.** Levels of ProLRP 1 in serum of patients with MDD (D) and controls (C). The levels of ProLRP1 dropped significantly (p <0.001) in serum of 45 patients with MDD and 21 healthy controls analysed by ELISA.
**Figure 6****.** Ratio of LRP1 / ProLRP1 levels in serum of patients with MDD (D) and controls (C) (Controls n = 21, MDD n = 45). It was determined by ELISA that the ratio LRP1/ProLRP1 are significantly elevated in MDD as compared with healthy controls (p <0.05).
**Figure 7****.** Ratio of LRP1/ProLRP1 levels in serum of patients with MDD stratified by sex. The figure shows how the LRP1/ProLRP1 ratio is significantly elevated in both males (p = 0.0104) and females (p = 0.0452) suffering from MDD as compared with healthy controls. CM (Control Male), DM (Depression Male), CF (Control Female) and DF (Depression Female).

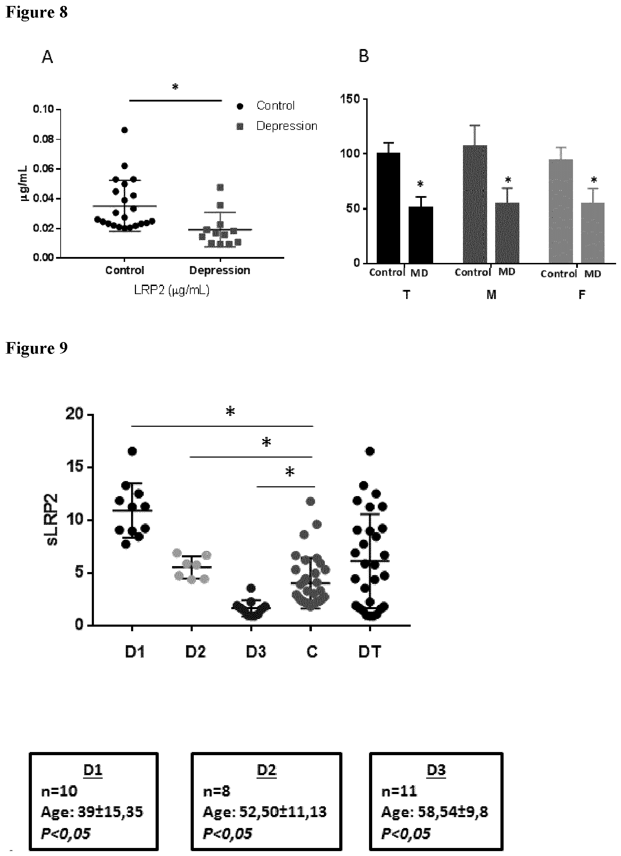

**Figure 8****.** Levels of soluble LRP2 (sLRP2 220 KDa) in serum of patients with MDD with ages between 50-60 years and controls. The quantification is carried out by ELISA. A) Graph of sLRP2 220 KDa levels in patients with MDD aged 45-60 years. The serum levels of sLRP2 decrease significantly (p <0.05) in MDD patients as compared with healthy controls. B) MDD is much more prevalent in women than in men, however, when the data is separated by sex, the variation of sLRP2 levels is the same in both groups. This indicates that sLRP2 could be used as a potential biomarker of MDD. T (Total). M (Male). F (Female).
**Figure 9****.** Patients are stratified by age and it is observed that there are 3 groups significantly different in terms of sLRP2 levels in serum: Depression 1 (D1) group comprises young patients with MDD and higher levels of sLRP2. Depression 2 (D2) comprises patients with MDD, having around 52 years old and levels of sLRP2 similar to the control. Depression 3 (D3) comprises patients with MDD, having around 58 years old and low levels of sLRP2 in serum. D3 is the group considered as pre-dementia, having a high probability of developing Alzheimer's disease. C (Control). DT (Total Depression).

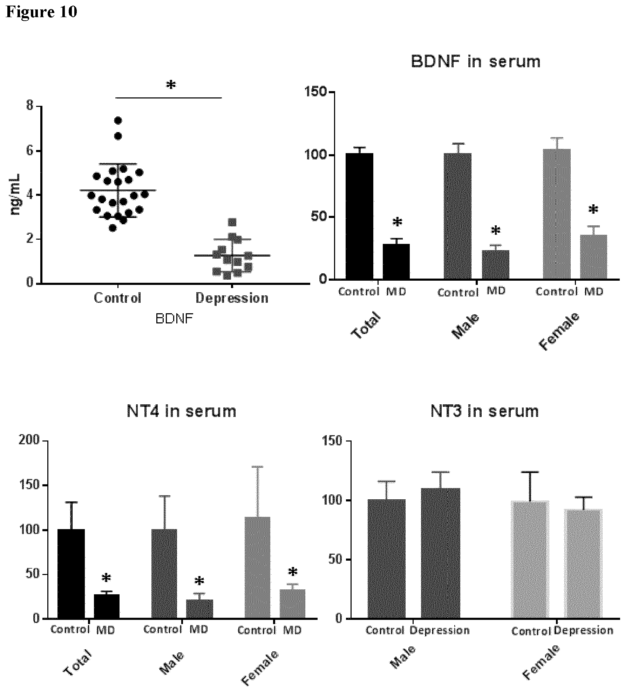

**Figure 10****.** Neurobiological characteristics of the patients with MDD assayed in the present invention. BDNF and NT4 are very clearly reduced in serum (p <0.05) of MDD patients.

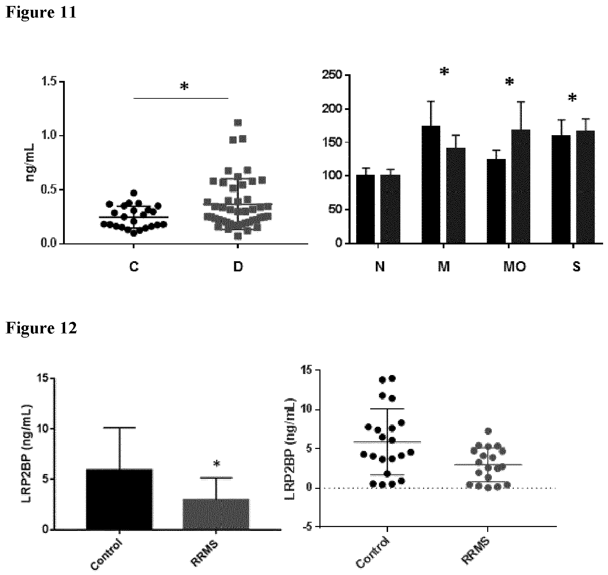

**Figure 11****.** Neurobiological characteristics of the group of patients with MDD assayed in the present invention. One of the characteristics of patients with MDD and Alzheimer's disease is that they have high levels of the neuroinflammatory protein lipocalin-2. Interestingly, it was determined that the group of patients with MDD of the present invention show elevated serum levels of lipocalin-2. When patients are stratified by severity (HAMD scale) and by intensity of anhedonia (SAAS scales) it remains elevated in all groups. C (Control). D (Depression). N (Normal). M (Mild). MO (Moderate). S (Serious).
**Figure 12****.** Levels of LRP2BP in CSF of patients with relapsing-remitting multiple sclerosis (RRMS). The level of LRP2BP decreases significantly in RRMS patients with respect to the control.

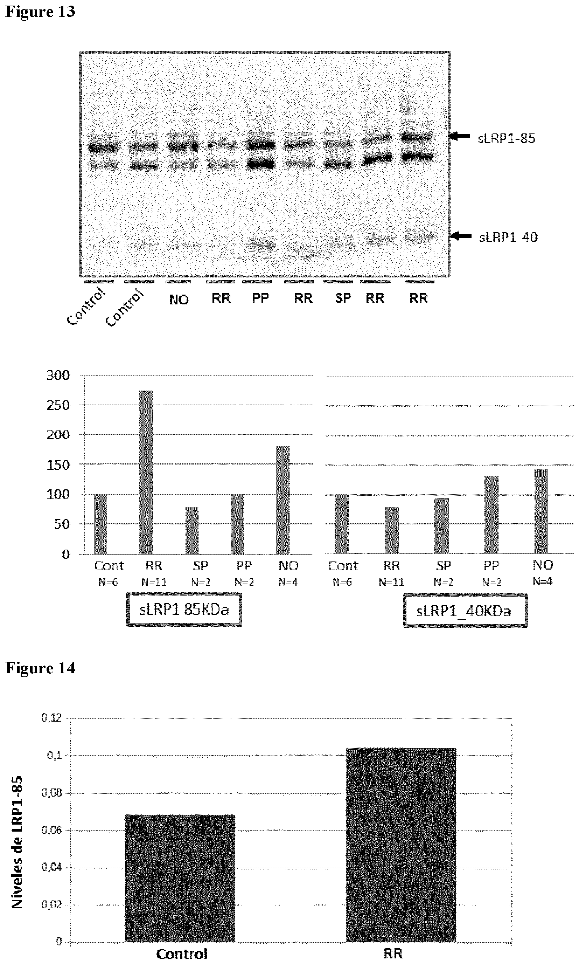

**Figure 13****.** Agarose gel of 9 samples of CSF (2 healthy controls, 1 patient with optic neuritis (NO), 4 patients with RRMS, 1 patient with SPMS and 1 patient with PPMS. It is observed that the fragment LRP1 85 KDa is able to distinguish the patients with RRMS from the rest. LRP1 85 KDa fragment is a potential biomarker of the EMRR. NO (optic neuritis). RR (Relapsing-Remitting MS). PP (Primary Progressive MS). SP (Secondary Progressive MS). MS (Multiple Sclerosis).
**Figure 14****.** Levels of sLRP1 85KDa in serum quantified by western blot densitometry with 18 control patients and 21 patients with naive RRMS. The levels of sLRP1 85 KDa are increased in the RRMS by 52% over the control. These data indicate that soluble LRP1 85 KDa in serum could be used as a biomarker of RRMS.

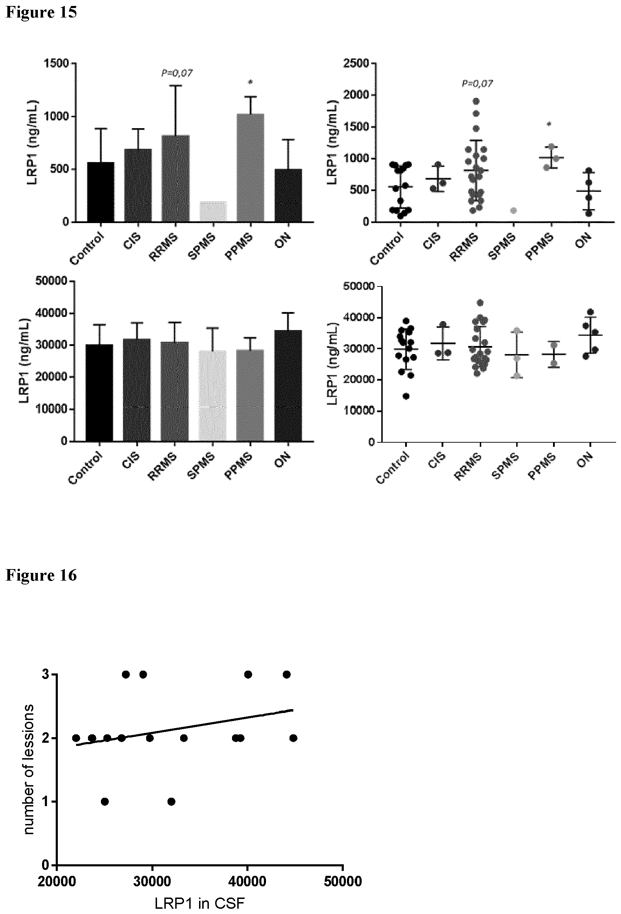

**Figure 15****.** Quantification of LRP1 levels measured by ELISA in serum (upper graphs) or in CSF (lower graphs). There are significant differences in patients diagnosed with RRMS and PPMS as compared with healthy controls. ON (optic neuritis). RR (Relapsing-Remitting MS). PP (Primary Progressive MS). SP (Secondary Progressive MS). Clinically Isolated Syndrome (CIS).
**Figure 16****.** This figure shows that there is a correlation between the number of lesions in the brain and the concentration of LRP1.

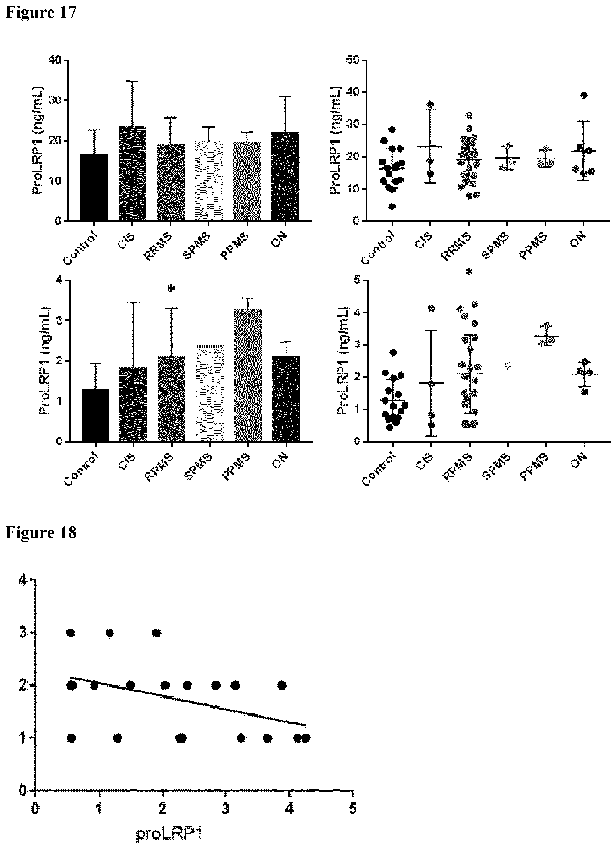

**Figure 17****.** Quantification of ProLRP1 levels measured by ELISA in serum (upper graphs) or in CSF (lower graphs). There are significant differences in patients diagnosed with RRMS. ProLRP1 can be used as a biomarker of neuroinflammatory disease. ON (optic neuritis). RR (Relapsing-Remitting MS). PP (Primary Progressive MS). SP (Secondary Progressive MS). Clinically Isolated Syndrome (CIS).
**Figure 18****.** A linear regression between the levels of ProLRP1 and the number of lesions in the brain shows correlation between the increase of the levels of ProLRP1 and the reduction of brain injuries.

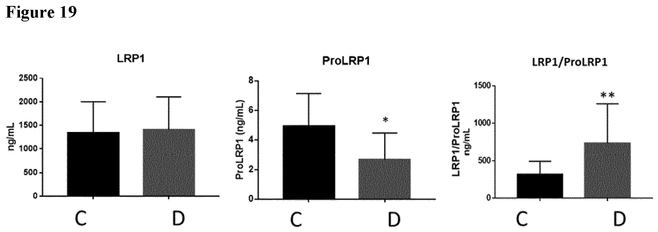

**Figure 19****.** Quantification of LRP1, pro-LRP1 and the ratio of LRP1/pro-LRP1 in control healthy patients (C) and patients suffering from major depression (D). The best results are obtained by the determination of the ratio LRP/pro-LRP1 and by the determination of pro-LRP1. However, the determination of LRP1 alone does not give rise to statistically significant results.

### EXAMPLES

### EXAMPLE 1. Material and Methods.

### Example 1.1. Human samples.

Venous blood was collected in vacuum tubes with K2EDTA between 7-9 a.m. from patients diagnosed with MDD and healthy controls. The samples of patients and controls were enrolled at Álvaro Cunqueiro Hospital, Vigo, Spain. Psychiatrists according to Diagnostic and Statistical Manual of Mental Disorders (DSM-V) diagnosed MDD patients. Inclusion criteria were MDD patients aged 18 years or older that provided the informed consent signed, agreeing with Helsinki Declaration and the approval of the Ethics Committee (Galician Network of Research Ethics Committees). Subjects with another psychiatric or neurological disorders, or traumatic brain injury were excluded, as well as, woman who are pregnant or breastfeeding. Patient group were matched by age and gender with healthy volunteers. Control group providing also the informed consent signed.

### Examples 1.2. Sample preparation.

Venous blood collected in two vacuum tubes with K2EDTA (BD vacutainer; Becton, Dickinson & Co., USA) was centrifuged at 1000xg for 10 min, and plasma was separated. After separation, plasma was stored in aliquots at -80°C. An aliquot of 450 µl of plasma were centrifuged at 16,000xg for 15 min at 4°C, recollecting and storing the supernatant at -80°C. Proteins were measured by BCA protein assay (Pierce Chemicals, Rockford, IL), and a protein enrichment commercial kit (ProteoMiner^{™}, Bio-Rad, Hercules, California, USA) was used according to manufacturer's instructions to equalize proteins in a sample with 10 mg of total proteins. Then, 10 µl of eluted fractions were loaded onto a standard Laemmeli-buffer 2× polyacrylamide gel and allowed to stack and enter the resolving gel, but not allowed separate. Manually, the gel was cut with a sterile scalpel in a flow chamber and the pieces of gel excised were subjected to in-gel digestion, which consisted on washing the gel pieces sequentially with ammonium bicarbonate 25 mM and 50% acetonitrile (ACN)/ammonium bicarbonate 25 mM, in ultrasonic bath. Subsequently, proteins were reduced with 10 mM dithiothreitol (DTT) for 1 hour, and alkylated with 55 mM indoleacetic acid (IAA) for 30 min. Finally, proteins were digested with 40 ng of trypsin at 37°C overnight and tryptic peptides were extracted from the gel matrix in 2 steps with 0.5% trifluoroacetic acid (TFA) and 100% ACN.

### Example 1.3. Western blot analyses.

Aliquots of plasma samples were prepared by addition of Laemmeli buffer 2x (Bio-Rad, California, USA) (950 µl of Laemmeli buffer and 50 µl of β-mercaptoethanol), and boiled at 95°C, 5 min. Plasma proteins previously measured (total proteins) allowed to determine the equivalent amount of proteins required for each sample (10-20 µg of proteins). Fraction samples were charged in 6-14% Bis-Tris polyacrylamide gels and electrophoresis was performed in PowerPac^{™} universal power supply (Bio-Rad, California, USA) at 60V for 30 minutes, and 120 V for 90 minutes. Proteins were immediately transferred to polyvinylidene difluoride (Immun-Blot^{®} PVDF membrane, Bio-Rad, California, USA), using PowerPac^{™} universal power supply (Bio-Rad, California, USA) at 0.25 A for 60 minutes (for two gels). Membranes were blocked with 5% milk in TBS-tween (TBST) for 20 minutes and they were washed three-times with TBST. Posteriorly, membranes were incubated overnight at 4°C in agitation with primary antibody: anti-LRP1, anti-Pro-LRP1, anti-LRP2 anti-LRP1 rabbit polyclonal antibody 1:2,500 (HPA022903, Sigma-Aldrich, St. Louis, USA), After washing three-times with TBST, membranes were incubated with the appropriate secondary antibody 1:10,000 (GE Healthcare Life Sciences, UK), during 60 minutes in agitation. Subsequently, membranes were washed two-times with TBST and one-time with TBS. ChemiDoc XRS+ system Bio-Rad, California, USA) was used to chemiluminescence analysis of membranes with ECL^{™} Prime Western Blotting System (Sigma-Aldrich, St. Louis, USA). Image Lab 6.0 software (Bio-Rad, California, USA) was used to blot images acquisition and densitometry band quantification was performed by ImageJ 1.5k software (National Institutes of Health, USA).

### Example 1.4. ELISA analysis.

We performed the ELISAs (LRP1, ProLRP, LRP2, BDNF, NT-3 and NT-4) following the indications of the manufacture.

### Example 1.5. In-gel digestion of proteins.

Protein samples containing 10 mg of total protein were enriched in Proteominer^{®} spin columns (Bio-Rad). Then 10 µL of eluents were loaded onto a standard Laemmli-type polyacrylamide gel and allowed to stack and enter the resolving gel, but not allowed to separate. Then, gel pieces were excised and subjected to in-gel digestion. Briefly, gel pieces were washed sequentially with ammonium bicarbonate 25 mM and 50% ACN/ammonium bicarbonate 25 mM, in an ultrasonic bath; proteins were reduced by treatment with 10mM DTT for 1 hour, and alkylated with 55mM IAA for 30 min. The digestion was accomplished with 40 ng of trypsin at 37 °C overnight and tryptic peptides were extracted from the gel matrix in two steps with 0,5% TFA and 100% ACN.

### Example 1.6. LC-MS/MS analysis.

The tryptic peptides were dried in a speed-vacuum concentrator at 45°C (Concentrator plus, Eppendorf, Hamburg, Germany), reconstituted in LC/MS-grade water containing 0.1% (v/v) formic acid and analysed by electrospray ionization-tandem mass spectrometry on a hybrid high-resolution LTQ-Orbitrap Elite mass spectrometer coupled to a Proxeon Easy-nLC 1000 UHPLC system (ThermoFisher Scientific).

The peptides were delivered onto a reverse phase column (PepMap^{®} RSLC C18, 2µm, 100 Å, 75µm × 50cm, Thermo Fisher Scientific) and were eluted with an ACN gradient of 5-30% containing 0.1% formic acid for 240 min at a flow rate of 5 µL/min. The elutes were directly delivered into the mass spectrometer, which was set in positive ion mode in a data-dependent manner. A full MS scan was performed from 380-1600 m/z with resolution at 120,000. The tandem mass spectrometer (MS/MS) scan was performed with top 15 at 38% normalized collision energy (NCE) with a dynamic exclusion time at 30 s, a minimum signal threshold at 1000, a resolution at 30,000, and an isolation width at 1.50 Da.

### Example 1.7. Identification and Quantification.

Proteome Discoverer (ThermoFisher Scientific) and PEAKS Studio v7.0 (Bioinformatics Solutions Inc.) programs were used for protein identification against NCBInr database from "Human", and for label-free relative quantification. A decoy sequence database was also included in the analysis to calculate the false discovery rate (FDR). Positive protein identifications were only accepted when the number of matched peptide sequences >2, unique peptides >1, peptide spectrum matches (PSMs) <0.5% FDR, and protein identification PEAKS score >20 (FDR 0.0%). Mass error tolerance and retention time shift tolerance were set at 20.0 ppm and 20.0 min, respectively, for label-free relative quantification.

### Example 1.8. Statistics.

Statistical analysis was performed using GraphPad Prism 5.0 (GraphPad Software Inc). All results are expressed as the mean ± standar desviation. Comparation between two groups was performed using paired or unpaired t-test.

### Example 2. Results.

### Example 2.1. LRP or fragments thereof as biomarkers for the diagnosis/prognosis of MDD.

**Figure 2** shows two gels of 8% western blot Acrylamide / Bisacrylamide where soluble fragments of LRP1 (right) and LRP2 (left) are identified. 3 fragments of LRP2 are identified in **Figure 2** left side: a fragment of 220KDa, a fragment of 86KDa and another fragment of 40KDa. Moreover, 2 fragments of LRP1 are identified in **Figure 2** right side: a fragment of 85KDa and a fragment of 48KDa. According to the AUC of the spectrum, LRP1 48 KDa is significantly lowered in the serum of patients with MDD (see **Figure 4**).

Next, the proteomic analyses were repeated with more patients and controls modifying parameters that would allow the detection of many more proteins in serum samples. Thus, **Figure 3** shows a heat-map graph of the LC-MS / MS analysis in the Orbitrap ELITE (Thermo Scientific) where another 4 serum samples, two control serum samples and 2 samples from patients were analysed in the context of MDD. 108 proteins were identified that differentiate between control subjects and subjects with depression. Among all these proteins, 7 fragments of LRP1 were identified that change their expression or concentration between both groups and are shown in **Figure 3**. Said fragments decrease their expression or concentration in the serum samples of patients with MDD.

In order to increase the sample size of both populations, levels of several fragments of LRP1 were measured using different ELISAs, one that specifically detects the levels of ProLRP1, a larger fragment of LRP1 and another ELISA that detects the overall levels of LRP 1.

**Figure 5** shows the levels of ProLRP1 in serum of patients with MDD and controls. The graph on the right side of **Figure 5** (proteomic analysis) shows how the levels of ProLRP1 measured by the AUC of the proteomic analysis spectrum decreases in patients with MDD as compared with the controls. When the levels are quantified by ELISA by means of the analysis of 45 patients with MDD and 21 healthy controls, the levels of ProLRP 1 dropped significantly in patients suffering from MDD (p <0.001).

Moreover, the ratio of LRP1 / ProLRP1 was quantified (**Figure 6**). When the ratio of serum LRP1 / ProLRP1 levels was analyses in patients with MDD (Controls n = 21, MDD n = 45), it was found that the ratio LRP1 / ProLRP1 is significantly elevated in patients with MDD (p <0.05).

In the same way as before, serum LRP1 / ProLRP1 levels in patients with MDD were compared according to their clinical severity values in depression (HAM-D scale) and anhedonia intensity (SAAS) (**Figure 7**). It is observed how the ratio LRP1 / ProLRP1 are significantly increased in both males (p = 0.0104) and females (p = 0.0452).

In conclusion, LRP1 through proteolytic processing releases soluble fragments that can be used as potential biomarkers to diagnose MDD. It is herein described for the first time the release of a new 48 KDa LRP1 fragment. After quantifying its levels by analysing the AUC of the LC-MS / MS it was observed how its levels are lowered in the serum of these patients. In addition, analysing the levels of ProLRP1, as well as the ratio LRP1 / ProLRP1, indicates that both biomarkers can be used for the diagnosis of MDD.

### Example 2.2. LRP or fragments thereof as biomarkers for the diagnosis/prognosis of Alzheimer disease.

Levels of LRP2 220 KDa was analysed in serum of patients with MDD with ages ranging from 45-60 and being diagnosed for the first time, since this subgroup has a high probability of developing Alzheimer's disease.

In order to validate this potential biomarker LRP2 220 KDa for the early diagnosis of Alzheimer's disease, a population at risk of developing Alzheimer disease was studied. Blood samples where obtained from patients diagnosed with MDD for the first time, with no previous history of MDD or other mental pathologies, ranging between 45 and 60 years old. It is considered that this population has a short-term risk to develop Alzheimer disease: mild cognitive impairment (MCI) in a first step and Alzheimer's disease afterwards.

**Figure 8** shows the levels of soluble LRP2 (sLRP2 220KDa) in serum of patients with MDD aged between 45-60 years old and controls measured by ELISA (reference). **Figure 8A** shows the total levels of LRP2-220 in 24 controls and 12 patients with MDD. Serum levels of LRP2-220 decrease significantly (p <0.05). **Figure 8B** shows stratification by sex. MDD is much more prevalent in women than in men, however, when data are split by sex, the variation of sLRP2 levels is equal in both groups. This indicates that LRP2-220 could be used as a potential biomarker of major depression pre-dementia.

In order to increase the study population, the levels in other patients diagnosed with MDD were measured. Said patients are younger, diagnosed for the first time with MDD and with no previous history of mental illness. **Figure 9** shows the levels of sLRP2 220 KDa in serum of patients with MDD and stratifying controls by age. It is observed that when the patients are stratified by age there are 3 significantly different groups in terms of serum LRP2 220 KDa levels:
- Group "Depression 1": Young patients with MDD and higher levels of sLRP2. These are the youngest individuals, with an average age of 45 years.
- Group "Depression 2": Patients with MDD and mean age of 52 years where the levels of LRP2-220 are similar to the control.
- Group "Depression 3": Patients with MDD and average age of 58 years that have low levels of LRP2 220 KDa in serum. Group 3 is considered as suffering pre-dementia in the present invention.

So, it can be concluded that within the patients diagnosed with MDD three groups are identified according to the expression or concentration level of LRP2 220 KDa and group 3 would be the potential group to develop dementia in the future.

Given that it is needed a minimum of 5 years to observe the evolution in order to validate this biomarker, a molecular study was performed based on what it is described in patients with MDD and in the patients with early diagnosis of Alzheimer's. It is known that these patients have low levels of neurotrophins in their blood, a family of proteins related to the development and functioning of the central nervous system. It is also known that in these patients the levels of BDNF and NT4 are very low and in NT3 levels there are no changes in these pathologies. So, the levels of these 3 neurotrophins were measured and it was observed that the above cited Group 3 has very low levels of BDNF and NT4 and we did not find changes with NT3 (see **Figure 10**).

Another characteristic of MDD and the early stages of Alzheimer's disease is that there is a neuroinflammatory process. Among the proteins related to inflammation there is one that has an important value, it is lipocalin 2 protein. This protein is described as being elevated in blood in both pathologies, and as shown in **Figure 11****,** it is observed that said protein is elevated in blood of the Group 3 of patients. This is an indication that they are facing a neuroinflammatory process. These data taken together support the fact that these patients are good candidates for the present study and that they could evolve towards the development of MCI and subsequent dementia. The fragments derived from the LRP1 and LRP2 receptors can be potential candidates to diagnose different neurodegenerative diseases such as MDD. The proposed candidates for the diagnosis of MDD are the combination of the determination of ProLRP1 on the one hand, and on the other, the ratio LRP1 / ProLRP1.

Finally, LRP2 220 KDa could be used as a biomarker for MDD in elderly patients with a high probability to develop Alzheimer's disease.

### Example 2.3. LRP or fragments thereof for the diagnosis/prognosis of MS.

LRP2BP (lipoprotein receptor-related protein-2-Binding Protein) is a 40KDa binding protein that interacts with LRP2.

When it was analysed a transport protein using LRP2, called LRP2BP, it was found that in CSF the levels of LRP2BP decrease in the different types of multiple sclerosis (MS) (**Figure 12**). These data indicate that LRP2BP in CSF can be a potential biomarker of the RRMS. They would have to increase the sample size to detect significant differences in the rest of the groups.

On the other hand, it is observed that the fragment LRP1 85 KDa is able to distinguish the patients with RRMS from the rest. LRP1 85 KDa fragment is a potential biomarker of the EMRR (**Figure 13**). Moreover, the levels of sLRP1 85 KDa are increased in the RRMS by 52% over the control. These data indicate that soluble LRP1 85 KDa in serum could be used as a biomarker of RRMS (**Figure 14**). On the other hand, the quantification of LRP1 levels measured by ELISA in serum or in CSF indicates that there are significant differences in patients diagnosed with RRMS and PPMS as compared with healthy controls (**Figure 15**). Moreover, there is a correlation between the number of lesions in the brain and the concentration of LRP1 (**Figure 16**). Moreover, the quantification of ProLRP1 levels measured by ELISA in serum or in CSF indicates that there are significant differences in patients diagnosed with RRMS. So, ProLRP1 can be used as a biomarker of neuroinflammatory disease (**Figure 17**). Finally, a linear regression between the levels of ProLRP1 and the number of lesions in the brain shows correlation between the increase of the levels of ProLRP1 and the reduction of brain injuries (**Figure 18**).

## Claims

1. *In vitro* method for the diagnosis or prognosis of major depression which comprises measuring, in a biological sample obtained from the patient, the level of expression or concentration level of LRP1 or a fragment thereof selected from the list comprising: LRP1 of SEQ ID NO: 9 and/or pro-LRP1 of SEQ ID NO:7, wherein if a deviation or variation of the ratio of the expression or concentration level of LRP1 of SEQ ID NO: 9 / proLRP1 of SEQ ID NO: 7, or of the level of expression or concentration level of pro-LRP1 of SEQ ID NO:7 is identified, as compared with the reference level of expression or concentration level measured in healthy control subjects, this is indicative that the subject is suffering from major depression or has a bad prognosis.

2. In *vitro* method, according to claim 1, which comprises measuring the ratio of the expression or concentration level of LRP1 of SEQ ID NO: 9 / proLRP1 of SEQ ID NO: 7 wherein if this ratio level is significantly higher as compared with the level measured in healthy controls, this is indicative that the subject is suffering from major depression or has a bad prognosis.

3. *In vitro* method, according to claim 1, which comprises measuring the level of expression or concentration level of pro-LRP1 of SEQ ID NO:7, wherein if the level of expression or concentration level is significantly lower as compared with the level of expression or concentration level measured in healthy controls, this is indicative that the subject is suffering from major depression or has a bad prognosis.

4. *In vitro* method, according to any of the previous claims, wherein the biological sample is serum.

5. In *vitro* use of the ratio of the expression or concentration level of LRP 1 of SEQ ID NO: 9 / proLRP1 of SEQ ID NO: 7, or of the level of expression or concentration level of pro-LRP1 of SEQ ID NO:7 for the diagnosis or prognosis of major depression.

6. Use of a kit comprising reagents for the detection of the ratio level of expression or concentration level of LRP1 of SEQ ID NO: 9/proLRP1 of SEQ ID NO: 7, or of the level of expression or concentration level of pro-LRP1 of SEQ ID NO: 7 for the diagnosis or prognosis of major depression.

## Patentansprüche

1. In-vitro-Verfahren zur Diagnose oder Prognose von schwerer Depression, welches das Messen, in einer biologischen Probe, welche aus dem Patienten erhalten wurde, des Expressionsniveaus oder Konzentrationsniveaus von LRP1 oder einem Fragment desselben ausgewählt aus der Liste umfassend: LRP1 aus SEQ ID NO: 9 und/oder pro-LRP1 aus SEQ ID NO:7, umfasst, wobei, wenn eine Abweichung oder Veränderung des Verhältnisses des Expressions- oder Konzentrationsniveaus von LRP1 aus SEQ ID NO: 9 / proLRP1 aus SEQ ID NO: 7, oder des Expressionsniveaus oder Konzentrationsniveaus von pro-LRP1 aus SEQ ID NO:7, im Vergleich zum Referenz-Expressionsniveau oder -Konzentrationsniveau identifiziert wird, welches in gesunden Kontrollindividuen gemessen wird, dies darauf schließen lässt, das das Individuum unter schwerer Depression leidet oder eine schlechte Prognose hat.

2. In-vitro-Verfahren nach Anspruch 1, welches das Messen des Verhältnisses des Expressions- oder Konzentrationsniveaus von LRP1 aus SEQ ID NO: 9 / proLRP1 aus SEQ ID NO: 7 umfasst, wobei, wenn dieses Verhältnisniveau bedeutend höher im Vergleich zum Niveau, welches in gesunden Kontrollindividuen gemessen wird, ist, dies darauf schließen lässt, dass das Individuum unter schwerer Depression leidet oder eine schlechte Prognose hat.

3. In-vitro-Verfahren nach Anspruch 1, welches das Messen des Expressionsniveaus oder Konzentrationsniveaus von pro-LRP1 aus SEQ ID NO: 7 umfasst, wobei, wenn das Expressionsniveau oder Konzentrationsniveau bedeutend niedriger im Vergleich zum Expressionsniveau oder Konzentrationsniveau, welches in gesunden Kontrollindividuen gemessen wird, ist, dies darauf schließen lässt, dass das Individuum unter schwerer Depression leidet oder eine schlechte Prognose hat.

4. In-vitro-Verfahren nach einem der vorhergehenden Ansprüche, wobei die biologische Probe Serum ist.

5. In-vitro-Verwendung des Verhältnisses des Expressions- oder Konzentrationsniveaus von LRP1 aus SEQ ID NO: 9 / proLRP1 aus SEQ ID NO: 7, oder des Expressionsniveaus oder Konzentrationsniveaus von pro-LRP1 aus SEQ ID NO:7 zur Diagnose oder Prognose von schwerer Depression.

6. Verwendung eines Kits, welches Reagenzien zur Detektion des Verhältnisses des Expressionsniveaus oder Konzentrationsniveaus von LRP1 aus SEQ ID NO: 9/proLRP1 aus SEQ ID NO: 7, oder des Expressionsniveaus oder Konzentrationsniveaus von pro-LRP1 aus SEQ ID NO: 7 zur Diagnose oder Prognose von schwerer Depression umfasst.

## Revendications

1. Procédé *in vitro* de diagnostic ou de pronostic de dépression majeure qui comprend la mesure, dans un échantillon biologique obtenu du patient, du niveau d'expression ou du niveau de concentration de LRP1 ou d'un fragment de celui-ci sélectionné de la liste comprenant : LRP1 de SEQ ID NO : 9 et/ou pro-LRP1 de SEQ ID NO : 7, dans lequel si une déviation ou une variation du rapport du niveau d'expression ou de concentration de LRP1 de SEQ ID NO : 9/pro-LRP1 de SEQ ID NO : 7, ou du niveau d'expression ou du niveau de concentration de pro-LRP1 de SEQ ID NO : 7 est identifiée, en comparaison avec le niveau de référence du niveau d'expression ou de concentration mesuré chez des sujets témoins sains, cela est révélateur que le sujet souffre de dépression majeure ou a un mauvais pronostic.

2. Procédé *in vitro,* selon la revendication 1, qui comprend la mesure du rapport du niveau d'expression ou de concentration de LRP1 de SEQ ID NO : 9/proLRP1 de SEQ ID NO : 7, dans lequel si ce rapport de niveau est significativement plus élevé en comparaison avec le niveau mesuré chez des témoins sains, cela est révélateur que le sujet souffre de dépression majeure ou a un mauvais pronostic.

3. Procédé *in vitro,* selon la revendication 1, qui comprend la mesure du niveau d'expression ou du niveau de concentration de pro-LRP1 de SEQ ID NO : 7, dans lequel si le niveau d'expression ou le niveau de concentration est significativement inférieur en comparaison avec le niveau d'expression ou le niveau de concentration mesuré chez des témoins sains, cela est révélateur que le sujet souffre de dépression majeure ou a un mauvais pronostic.

4. Procédé *in vitro,* selon l'une quelconque des revendications précédentes, dans lequel l'échantillon biologique est du sérum.

5. Utilisation *in vitro* du rapport du niveau d'expression ou de concentration de LRP1 de SEQ ID NO : 9/pro-LRP1 de SEQ ID NO : 7, ou du niveau d'expression ou du niveau de concentration de pro-LRP1 de SEQ ID NO : 7 pour le diagnostic ou le pronostic de dépression majeure.

6. Utilisation d'un kit comprenant des réactifs pour la détection du rapport de niveau d'expression ou du niveau de concentration de LRP1 de SEQ ID NO : 9/proLRP1 de SEQ ID NO : 7, ou du niveau d'expression ou du niveau de concentration de pro-LRP1 de SEQ ID NO : 7 pour le diagnostic ou le pronostic de dépression majeure.
